# EUROPEAN PATENT SPECIFICATION

(11) **EP 0 781 280 B1**
(45) Date of publication and mention of the grant of the patent: **06.11.2002**
(21) Application number: 95931987.2
(22) Date of filing: 05.09.1995
(51) Int. Cl.: C07D 417/12, C07D 311/64, C07D 335/06, A61K 31/425, A61K 31/35, A61K 31/38

(54) **DERIVATIVES OF BENZOPYRAN AND OF BENZOTHIOPYRAN, THEIR PREPARATION AND THEIR USE AS AGONISTS OF DOPAMINERGIC RECEPTORS**
DERIVATE DES BENZOPYRANS UND DES BENZOTHIOPYRANS, DEREN HERSTELLUNG UND DEREN VERWENDUNG ALS AGONISTEN VON DOPAMIN REZEPTOREN
DERIVES DE BENZOPYRANE ET DE BENZOTHIOPYRANE, LEUR PREPARATION AINSI QUE LEUR UTILISATION EN TANT QU'AGONISTES DES RECEPTEURS DOPAMINERGIQUES

(30) Priority: 13.09.1994 IT MI941870
(43) Date of publication of application: 02.07.1997
(73) Proprietor: ZAMBON GROUP S.p.A., 36100 Vicenza (IT)
(72) Inventor: NAPOLETANO, Mauro, I-20127 Milano (IT); CAVALLERI, Paolo, I-20144 Milano (IT); FRAIRE, Cristina, I-20025 Legnano (IT); GRANCINI, Gian, Carlo, I-20054 Nova Milanese (IT); MONTANARI, Stefania, I-20139 Milano (IT); SANTANGELO, Francesco, I-20148 Milano (IT)
(86) International application number: EP9503478
(87) International publication number: WO96008489

(56) References cited:
- EP-A- 0 280 269
- EP-A- 0 321 968
- WO-A-90/12795
- WO-A-93/19036
- WO-A-95/07885
- JOURNAL OF MEDICINAL CHEMISTRY, vol. 31, no. 3, March 1988 pages 688-691, WISE L.D. ET AL. '6- and 8-hydroxy-3,4-dihydro-3-(dipropylamino)-2H -1-benzopyrans. Dopamine agonists with autoreceptor selectivity'
- JOURNAL OF MEDICINAL CHEMISTRY, vol. 31, no. 11, November 1988 pages 2178-2182, DIJKSTRA D. ET AL. 'Synthesis and pharmacology of trans-4-n-propyl-3,4,4a,10b-tetrahydro-2H. 5H-1-benzopyrano[4,3-b]-1,4-oxazin-7- and -9-ols: The significance of nitrogen pKa values for central dopamine receptor activation'

## Description

The present invention relates to compounds active on the cardiovascular system and, in particular, it relates to derivatives of benzopyran and of benzothiopyran and to their use in therapeutic field.

It is known that among the dopamine analogs several cyclization compounds such as, for example, the derivatives of 2-amino-1,2,3,4-tetrahydro-naphthalene and their isosters 3-amino-3,4-dihydro-2H-1-benzopyrans and benzothiopyrans retain a significant dopaminergic activity.

For example, Wise L.D. et al., J. Med. Chem. 31(3), 688-691 (1988), describes benzopyran derivatives showing a direct agonist activity at brain dopamine receptors, EP-A-0280269 (Ciba Geigy AG) describes benzopyran and benzothiopyran derivatives useful as modulators for the receptors of the CNS and WO 90/12795 (The Upjonh Company) describes chromane derivatives useful to treat CNS disorders.

In contrast, Dijkstra D. et al., J. Med. Chem. 31(11), 2178-2182 (1988) reports that 6-oxa analogs of potent dopamine agonists were found to be almost inactive.

On the dopamine analogs, several studies about the structure-activity relationship have been carried out to determine the structural characteristics able to ensure the best dopaminergic activity and to avoid, at the same time, the undesired effects of dopamine.

An interesting review of these studies is collected in a paper published by H.E. Katerinopoulos and D.I. Schuster on Drugs of the Future, vol. 12(3), pages 223-253, (1987).

In spite of the various studies, however, the topology of dopaminergic receptors has not yet been explained and a series of receptor models has been proposed in the last ten years.

In the field of the compounds structurally related to dopamine, some authors have found that the presence of a C₃-C₄ alkyl group on the amino function is one of the requirements for dopaminergic activity while the structural requirements of the second substituent on the amino group have not yet been found.

Nevertheless, there are several examples in literature showing how the structural features of the two substituents can be, in practice, extremely variable and how small changes of the molecule can affect the pharmacological activity both quantitatively and qualitatively in a relevant manner.

Among the most significant examples the following are cited.

European patent application no. 72061 (Fisons PLC) describes, among the others, dopamines and aminotetrahydronaphthalenes having a mono- or di-substituted portion of formula 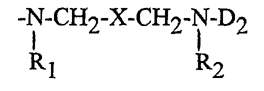 wherein
X is a -(CH₂)ₙ- chain, optionally substituted by hydroxies; n is an integer between 1 and 7; R₁ and R₂, the same or different, are hydrogen, alkyl or phenyl; D₂ is hydrogen, alkyl, phenyl; alkyl substituted by one or more hydroxies, pyridyls, phenyls; alkyl substituted by phenyl substituted, in turn, by halogen, alkyl, amino, alkoxy or nitro; or D₂ may be an optionally mono- or di-hydroxy substituted phenylethyl or tetrahydronaphthyl moiety.

Among the compounds described in European patent application No. 72061, the compound of formula 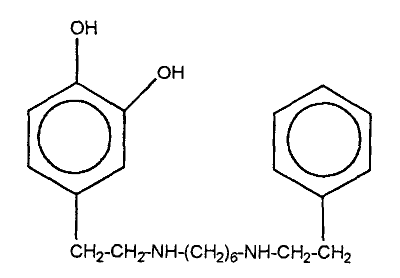 whose International non-proprietary name is dopexamine (The Merck Index - XI ed., No. 3418, page 538) is the only compound, as far as we know, which has been developed and used in the acute treatment of failure.

It is significant that dopexamine, notwithstanding it was selected among the several compounds described and exemplified in European patent application no. 7206l is an agonist of dopaminergic receptors less active than dopamine and, like dopamine itself, it is not absorbed when administered by oral route [A. Fitton and P. Benfield, Drugs, 39(2), 308-330, (1990)].

European patent application no. 142283 (Fisons PLC) describes a class of compounds which are analogs of dopexamine and in which the amino group of the dopamine moiety is still secondary.

In literature, there are several compounds with a catecholamine structure having the aim of keeping the favorable properties of dopexamine, also when administered by oral route, or of increasing the selectivity towards the dopaminergic receptors.

Among these, those described in European patent application No. 321968 (SIMES Società Italiana Medicinali e Sintetici S.p.A.) having the following general formula: 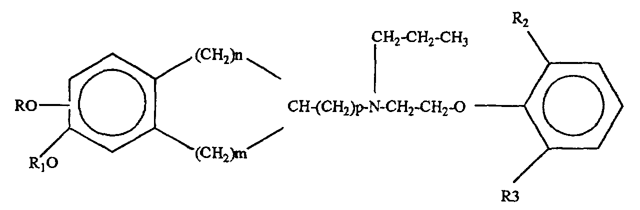 wherein
R and R₁, the same or different, are hydrogen or acyl deriving from an aliphatic, aromatic or heteroaromatic carboxylic acid, from a carbonic or carbamic acid or from a phosphoric acid; n and p are integer numbers selected among 0 and 1; m is an integer number selected among 1, 2, 3 and 4 so that n+p=1 and m+n is 2, 3 or 4; R₂ and R₃, the same or different, are hydrogen, halogen, alkyl or alkoxy;
are particularly interesting.

These compounds are agonists of D₁ and D₂ dopaminergic receptors, show contemporaneously an ∝₁-antagonist effect, do not interact with other receptor systems, but in order to be active by oral administration they have to be transformed into suitable pro-drugs.

The compounds described in the International patent application WO 93/19036 (Zambon Group S.p.A.), which are dopaminergic agonists more potent than dopamine and not selective towards any specific receptor sub-type, which do not interact with other receptor systems and which, at the same time, do not show either the side effect or the therapeutically disadvantageous aspects of dopamine, are still more interesting.

The compounds described in the above cited International patent application have the following general formula: 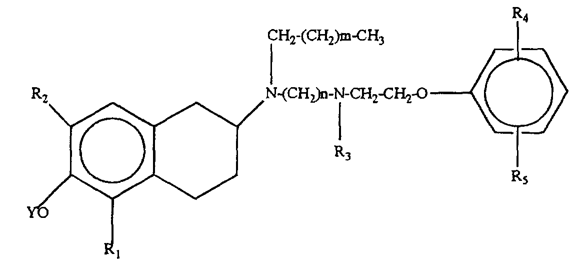 R₁ and R₂, the same or different, are hydrogen atoms or OY' groups; Y and Y', the same or different, are hydrogen atoms or acyl groups deriving from an aliphatic, aromatic or heteroaromatic carboxylic acid, from a carbonic or carbamic acid or from a phosphoric acid; m is 1 or 2; n is an integer number among 3 and 7; R₃ is hydrogen or C₁-C₄ alkyl; R₄ and R₅, the same or different, are hydrogen, halogen, C₁-C₃ alkyl or alkoxy.

Dopaminergic agonists more potent than dopamine but without its side-effects or therapeutic disadvantages are described also in the International patent application WO 95/07885 (Zambon Group S.p.A.).

These compounds have the following formula: 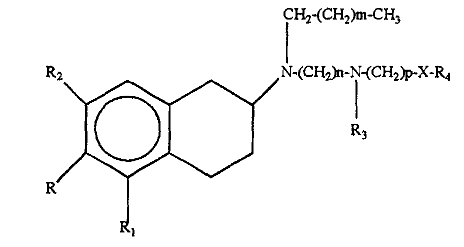 wherein
R is a hydrogen atom or an OY group; R₁ is a hydrogen atom or an OY' group; R₂ is a hydrogen atom or an OY" group; provided that at least one among R, R₁ and R₂ is hydrogen but R, R₁ and R₂ are not contemporaneously hydrogen atoms and R₁ and
R₂ are not contemporaneously OY' or OY" groups respectively; Y, Y' and Y", the same or different, are hydrogen atoms or acyl groups; m is 1 or 2; n is 3-8; p is 2-4; R₃ is a hydrogen atom or a C₁-C₄ alkyl; R₄ is an optionally substituted phenyl or heteroaryl; X is CH₂, NH, S, SO, SO₂, CO, CF₂, O or a single bond.

However, as far as we know, in the specific field of benzopyran and benzothiopyran derivatives, compounds having an interesting dopaminergic activity useful in the cardiovascular field, particularly for the treatment of arterial hypertension and heart failure, of renal failure, in the treatment of peripheral arteriopathies, of cerebrovascular insufficiencies and of ischemic cardiopathy have been never described up to now.

We have now found derivatives of benzopyran and of benzothiopyran which are agonists of dopaminergic receptors more potent than dopamine and which are particularly useful in cardiovascular field.

Therefore, object of the present invention are compounds of formula 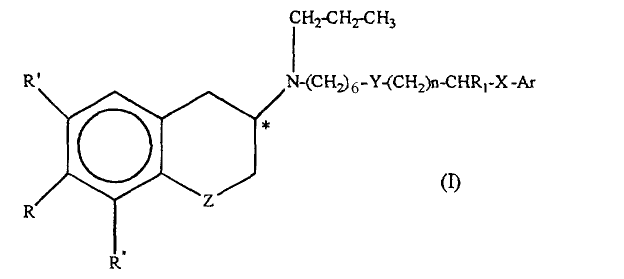 wherein
- Z: is O, S, SO or SO₂;
- R, R' and R": are hydrogen atoms or OH groups, provided that at least one among
- R, R' and R": is a hydrogen atom but R, R' and R" are not all contemporaneously hydrogen atoms and R' and R" are not both contemporaneously OH groups;
- n: is an integer number selected among 0, 1, 2, 3 and 4;
- R₁: is a hydrogen atom or a C₁-C₄ alkyl group;
- Ar: is a phenyl of formula 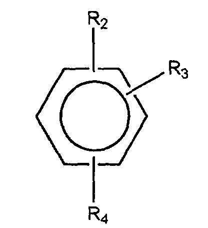 wherein
R₂, R₃ and R₄, the same or different, are hydrogen, OH, halogen, C₁-C₄ alkyl, C₁-C₄ alkoxy, nitro, C₁-C₄ alkylthio, NH₂, mono- or di-C₁-C₄ alkylamino, SH, C₁-C₄ alkylsulphonyl, C₁-C₄ alkoxycarbonyl, NHCHO, C₁-C₄ alkylcarbonylamino, NHCONH₂, C₁-C₄ alkylsulphonylamino, C₁-C₄ alkylaminosulphonyl, SO₂NH₂, NHSO₂NH₂, COOH, SO₃H, CONH₂, CH₂OH or phenyl; or
R₂ and R₃, in ortho position one with respect to the other, together form an optionally unsaturated chain made by 3 or 4 groups selected among CR"'R^{IV}, CO, S, O and NR^{V}, wherein R"' is a hydrogen atom or a C₁-C₄ alkyl group, R^{IV} is a hydrogen atom, a C₁-C₄ alkyl group or an amino group and R^{V} is a hydrogen atom or a C₁-C₄ alkyl group; or R"' together with a vicinal R"' or R^{V} constitutes a single bond or R^{V} together with a vicinal R"' or R^{V} constitutes a single bond;
- Y: is S, O, N(H)CO, CO(H)N or N(H);
- X: is N(H), O, S, SO, SO₂, CO or a single bond;
the asterisk marks an asymmetric carbon atom;
and pharmaceutically acceptable salts thereof.

The compounds of formula I have at least an asymmetric center, marked by an asterisk, and they may be in the form of stereoisomers.

Object of the present invention are the compounds of formula I in the form of stereoisomeric mixtures as well as in the form of single stereoisomers.

The compounds of formula I are agonists of dopaminergic receptors active also by oral route and with long duration of action and they are useful in therapy in cardiovascular field, in particular for the treatment of arterial hypertension and heart failure, of renal failure, in the treatment of peripheral arteriopathies, of cerebrovascular insufficiencies and of ischemic cardiopathy.

With the term halogen atom we intend a fluorine, chlorine, bromine or iodine atom.

Specific examples of alkyl or alkoxy groups are methyl, ethyl, n.propyl, i.propyl, n.butyl, i.butyl, sec.butyl, t.butyl, methoxy, ethoxy, n.propoxy, i.propoxy, n.butoxy and i.butoxy.

Specific examples of optionally unsaturated chains made by 3 or 4 groups selected among CR'"R^{IV}, CO, S, O and NR^{V} are the following: -O-CHR"'-O-, -S-CO-NR^{V}-, -CHR"'-CO-NR^{V}-, -S-CR^{IV}=N-, -O-CO-NR^{V}-, -CO-NR^{V}-NR^{V}-, -NR^{V}-CO-NR^{V-}

Preferred compounds of formula I are the compounds wherein R' is a hydrogen atom, R and R" are OH groups.

Still more preferred compounds are the compounds wherein R' is a hydrogen atom, R and R" are OH groups.

Among the meanings of the substituents R₂, R₃ and R₄, the preferred are hydrogen, OH, methoxy, methyl, nitro, chloro, methylsulphonyl, NH₂, SO₂NH₂, methylsulphonylamino, NH₂CONH₂, methoxycarbonyl, acetylamino, CONH₂, CH₂OH and SO₃H or, when R₂ and R₃ in ortho position one with respect to the other form a chain, a methylendioxy group or a group of formula -S-CO-NR^{V}- wherein R^{V} is a hydrogen atom.

Still more preferred among the meanings of R₂, R₃ and R₄ are OH, methoxy and -S--CO-NR^{V}- wherein R^{V} is a hydrogen atom.

Pharmaceutically acceptable salts of the compounds of formula I are the salts with organic and inorganic acids such as, for example, hydrochloric, hydrobromic, hydroiodic, nitric, sulphuric, phosphoric, acetic, benzoic, maleic, fumaric, succinic, tartaric, citric, aspartic, methansulphonic and 3,7-di-tertbutylnaphthalen-1,5-disulphonic acid (dibudinic acid).

The preparation of the compounds of formula I can be carried out according to the synthetic method described herein after.

The method comprises the reaction between a compound of formula 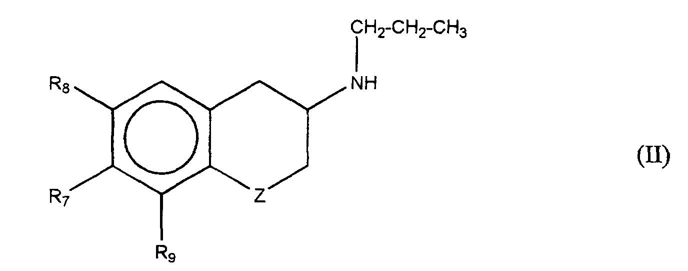 wherein
- Z: has the already reported meanings;
- R₇, R₈ and R₉: are hydrogen atoms or OA groups wherein A is a hydrogen atom or a protective group selected, for example, among methyl, benzyl, benzoyl and 4-methoxybenzoyl, provided that at least one among R₇, R₈ and R₉ is a hydrogen atom but R₇, R₈ and R₉ are not all contemporaneously hydrogen atoms and R₈ and R₉ are not both contemporaneously OA groups;
and an acid of formula 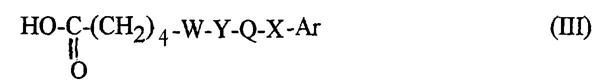 wherein, X, Y and Ar have the already reported meanings; W is a CH₂ or CO group; Q is a group of formula

CO-(CH₂)ₙ₋₁-CHR₁

wherein R₁ has the already reported meanings, when n is 1, 2, 3 or 4; or Q is a CHR₁ or CO group, wherein R₁ has the already reported meanings, when n is 0;
or a reactive derivative thereof such as an acyl halide or a mixed anhydride which can optionally be prepared in situ, in an inert solvent and in the presence of a base such as an alkaline carbonate or bicarbonate or a tertiary amine,
in order to obtain the intermediate compounds of formula 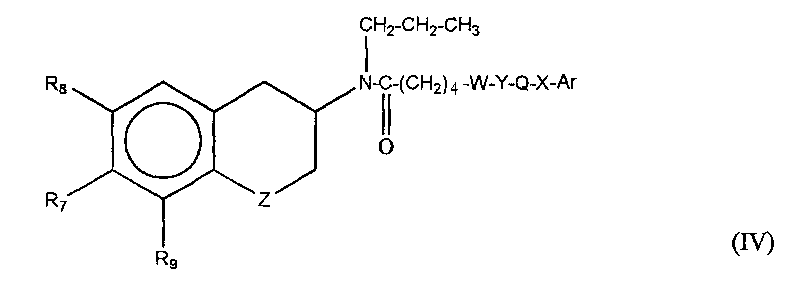 wherein Ar, Z, W, Y, Q, X, R₇, R₈ and R₉ have the already reported meanings; and their reduction, preceded or followed by the optional deprotection of the hydroxy groups, in order to obtain the compounds of formula I.

The reduction of the compounds of formula IV can be carried out with electrophile reducing agents, in particular with diborane optionally complexed with dimethylsulphide, tetrahydrofuran, aliphatic amines such as triethylamine or aromatic amines such as N,N-diethylaniline or pyridine.

Alternatively, the reduction can be carried out with nucleophile reducing agents such as metal hydrides, for example lithium aluminum hydride.

The reduction reaction is carried out in a suitable solvent such as for example tetrahydrofuran, diethylether or 1,2-dimethoxyethane.

The optional deprotection of the hydroxy groups is carried out according to conventional techniques such as hydrolysis or hydrogenolysis.

The compounds of formula II are known or can be prepared according to known methods [Al Neirabeyeh M. et al., Eur. J. Med. Chem. (1991) 26, 497-504].

Also the compounds of formula III are known or easily prepared according to conventional methods.

Alternatively, the compounds of formula I wherein Y is a N(H) group can be prepared following a different sequence.

At first it is carried out the condensation reaction between a compound of formula II and a suitable reactive derivative of an acid of formula 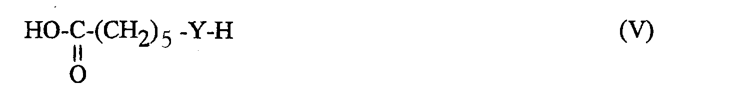 wherein Y is an N(H) group; followed by the optional reduction according to what already reported,
in order to obtain the intermediate of formula 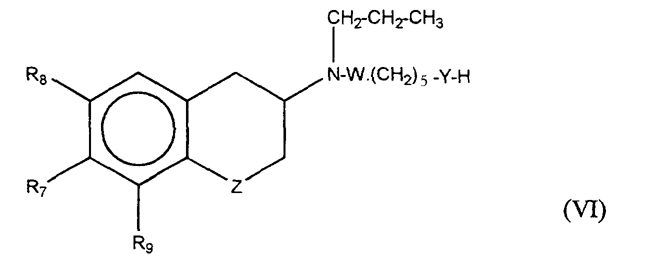 wherein Y is an N(H) group; Z, W, R₇, R₈ and R₉ have the already reported meanings.

The intermediate of formula VI is then reacted with a suitable reactive derivative of an acid of formula 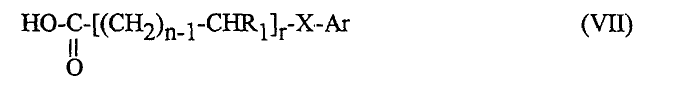 wherein n, R₁, Ar and X have the already reported meanings; r is 0 or 1;
obtaining the corresponding intermediates of formula IV.

The subsequent reduction, preceded or followed by the optional deprotection of the hydroxy groups, gives the compounds of formula I wherein Y is a N(H) group, object of the present invention.

A further alternative for the preparation of the compounds of formula I wherein Y is an N(H) group consists in first reacting a compound of formula II with a suitable reactive derivative of a bicarboxylic acid of formula 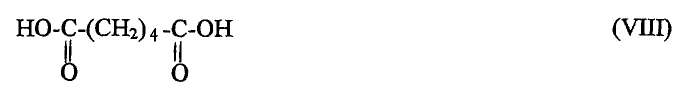 in order to obtain an intermediate of formula 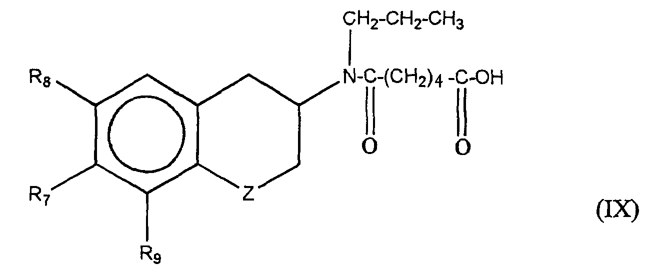 wherein Z, R₇, R₈ and R₉ have the already reported meanings.

The subsequent condensation with an amine of formula

H-Y-(CH₂)ₙ-CHR₁-X-Ar (X)

wherein Y is an N(R₅) group; n, X, R₁ and Ar have the already reported meanings;
gives the corresponding intermediates of formula IV.

The subsequent reduction, preceded or followed by the optional deprotection of the hydroxy groups, allows to obtain the compounds of formula I, object of the present invention.

Some of the compounds of formula I, optionally protected, may also be used as intermediates for the synthesis of other compounds of formula I by transformation of certain functional groups present in the molecule.

Thus, for example, compounds of formula I wherein one or more of R₂, R₃ and R₄ is a nitro group or an alkoxycarbonyl group can be transformed into the corresponding compounds of formula I wherein one or more R₂, R₃ and R₄ is an amino or carboxy group by reduction or hydrolysis respectively. In turn, compounds of formula I wherein one or more of R₂, R₃ and R₄ is an amino group or a carboxy group can be transformed into the corresponding compound of formula I wherein one or more of R₂, r₃ and R₄ is an alkylcarbonylamino or CH₃OH gorup by acylation or reduction respectively.

Analogously, the same transformation can be carried out on intermediates of formula IV.

The compounds of formula I in optically active form are obtained by optical separation or by stereospecific or stereoselective synthesis.

The preparation of salts of the compounds of formula I is carried out according to conventional methods.

The compounds of formula I are agonists of D₁ and D₂ dopaminergic receptors having more affinity than dopamine and than dopexamine as results from the in vitro binding tests.

Furthermore, their activity is at least comparable to that of the compounds described in the alreaxy cited International patent application WO 93/19036.

The in vitro results have been confirmed also by functional studies on isolated tissues, which are predictive of the in vivo activity, such as the Rabbit Splenic Artery (RSA) test and the Rabbit Ear Artery (REA) test (example 15).

The tests for interaction with the other receptor systems showed that the compounds of formula I do not significantly interact and then are endowed with high specificity.

The compounds of formula I resulted also to be inactive on the central nervous system after oral administration and this lack of effect is a further positive property generally not shared with the other compounds with cathecolamine structure.

It is clear how these characteristics of selectivity and receptor specificity together with the lack of activity on the central nervous system make the compounds of formula I particularly suitable for the treatment of cardiovascular diseases and mainly in the antihypertensive therapy, in the therapy of hearth failure, of renal insufficiency, in the treatment of peripheral arteriopathies, of cerebrovascular insufficiencies and of ischemic cardiopathy.

In addition to the already underlined higher pharmacologic activity, the feature which more characterizes the compounds of formula I, object of the invention, is their absorbability by oral route and their prolonged duration of action.

As a consequence for the practical uses in therapy, the compounds of formula I can be administered by parenteral route as well as by enteral route so differing from dopamine and from dopexamine.

The therapeutic doses will be generally comprised between 5 mg and 1 g a day and between 1 and 300 mg by oral route for each single administration.

The pharmaceutical compositions containing a therapeutically effective amount of the compounds of formula I or of their pharmaceutically acceptable salts in admixture with a suitable carrier are, furthermore, object of the present invention.

The pharmaceutical compositions object of the invention may be liquid, suitable for enteral or parenteral administration, and, preferably, solid such as tablets, capsules, granulates, suitable for oral administration.

The preparation of the pharmaceutical compositions object of the invention can be carried out according to traditional techniques.

Notwithstanding the compounds of formula I are active as such also when orally administered, in order to fulfill some specific therapeutic or pharmaceutical requirements it can be useful to transform them into the corresponding pro-drugs.

According to the techniques used in the field of phenolic and cathecolic derivatives, suitable pro-drugs are obtained by esterification of one or two hydroxy groups with pharmaceutically acceptable salts.

Specific examples of pro-drugs of the compounds of formula I are acetoxy derivatives, wherein the hydroxy groups are esterified with acetic acid, and mono- or diphosphonates, wherein one or both hydroxy groups are esterified with phosphoric acid.

The compounds of formula I also when transformed into pro-drugs and in particular the compounds obtained by esterification of the phenolic hydroxy groups or of one or both the cathecolic hydroxy groups with pharmaceutically acceptable acids, as well as the pharmaceutical compositions which contain a compound of formula I in the form of a corresponding pro-drug, and in particular which contain a compound of formula I wherein the phenolic hydroxy groups or of one or both the cathecolic hydroxy groups are esterified with pharmaceutically acceptable acids are within the scope of the present invention.

In order to better illustrate the present invention the following examples are now given.

The chromatographic purifications were carried out on silica gel (230-400 mesh) columns.

The mass spectra were carried out under the following conditions: chemical ionization, isobutane, positive ions.

### Example 1

### Preparation of 2,3-dimethoxybenzenethiol

A solution of n.butyl lithium in hexane (67 ml; 0.167 moles) was gently added to a cooled (-20°C) solution of 1,2-dimethoxybenzene (14.2 ml; 0.11 moles) and tetramethylethylendiamine (25 ml; 0.167 moles) in anhydrous tetrahydrofuran (100 ml), under nitrogen atmosphere.

The mixture was stirred at room temperature for 1 hour, cooled at -60°C and treated with recrystallized sulphur (5.36 g; 0.167 moles), added in one portion.

The reaction mixture, after stirring at room temperature overnight, was quenched with aqueous potassium carbonate (100 ml) and extracted with ethyl ether (100 ml).

The aqueous phase was acidified with hydrochloric acid (pH about 1) and re-extracted twice with ethyl acetate.

The collected organic layers were dried on anhydrous sodium sulphate and evaporated to afford an orange oil which was purified by flash chromatography (eluent hexane:ethyl acetate=98:2) obtaining 2,3-dimethoxybenzenethiol (9.7 g; 51% yield) as a yellow oil.

¹H-NMR (200 MHz, CDCl₃): δ (ppm): 6.96-6.64 (m, 3H); 3.87-3.82 (m, 7H).

Mass: 171 [M+1].

### Example 2

### Preparation of 2-[[(2.3-dimethoxyphenyl)thio]methyl]propenoic acid

By working according to the method described on page 47 of European patent application No. 0 161 218 (Ciba-Geigy AG), 2-[[(2,3-dimethoxyphenyl)thio]methyl]propenoic acid (92% yield) was obtained as a yellow oil.

¹H-NMR (200 MHz, CDCl₃): δ (ppm): 10.0 (bs, 1H); 7.02-6.75 (m, 3H); 6.29 and 5.72 (2bs, 2H); 3.86-3.83 (2s, 6H); 3.78 (s, 2H).

Mass: 255 [M+1].

Analogously the following compound was prepared.

2-[[(2-methoxyphenyl)thio]methyl]propenoic acid
having physico-chemical characteristics corresponding to those described in the European patent application No. 0 161 218.

### Example 3

### Preparation of 3,4-dihydro-7,8-dimethoxy-1(2H)-benzothiopyran-3-carboxylic acid

By working according to the method described on page 47 of European patent application No. 0 161 218 (Ciba-Geigy AG), 3,4-dihydro-7,8-dimethoxy-1(2H)-benzothiopyran-3-carboxylic acid (73% yield) was obtained as a yellow solid.

¹H-NMR (200 MHz, CDCl₃): δ (ppm): 6.80 (d, 1H); 6.61 (d, 1H); 3.83 and 3.81 (2s, 6H); 3.21-2.90 (m, 5H).

Mass: 255 [M+1].

Analogously the following compound was prepared.

3,4-dihydro-8-methoxy-1(2H)-benzothiopyran-3-carboxylic acid
having physico-chemical characteristics corresponding to those described in European patent application No. 0 161 218.

### Example 4

### Preparation of 3,4-dihydro-7,8-dimethoxy-1(2H)-benzothiopyran-3-amine hydrochloride

A solution of 3,4-dihydro-7,8-dimethoxy-1(2H)-benzothiopyran-3-carboxylic acid (9.5 g; 0.037 moles), prepared as described in example 3, in distilled t.butanol (60 ml) was stirred at room temperature, under nitrogen atmosphere.

Diphenylphosphorylazide (8.9 ml; 0.041 moles) and distilled triethylamine (5.72 ml; 0.041 moles) were added and the solution was heated under reflux for 19 hours.

The formation and the disappearance of the isocyanate infrared peak (2254 cm⁻¹) were used to monitor the reaction.

The solvent was removed under vacuum and the residue was taken up with ethyl acetate (200 ml), washed with aqueous sodium bicarbonate, followed by hydrochloric acid (2.5%) and brine.

The organic layers were dried on anhydrous sodium sulphate and concentrated under reduced pressure to give the N-[[(1,1-dimethyl)ethoxy]carbonyl]amine intermediate (13 g) as a yellow oil.

This raw intermediate was dissolved in ethyl acetate saturated with hydrochloric acid (100 ml) and stirred overnight to afford, after filtration, 3,4-dihydro-7,8-dimethoxy-1(2H)-benzothiopyran-3-amine hydrochloride (63% yield) as a white solid.

¹H-NMR (200 MHz, DMSO-d₆): δ (ppm): 8.45 (bs, 3H); 6.87 (d, 1H); 6.78 (d, 1H); 3.87 and 3.79 (2s, 6H); 3.70-3.56 (m, 1H); 3.22-2.79 (m, 4H).

Mass: 226 [M+1].

By working in a similar way the following compound was prepared.

3,4-dihydro-8-methoxy-1(2H)-benzothiopyran-3-amine hydrochloride white solid.

¹H-NMR (200 MHz, DMSO-d₆): δ (ppm): 8.50 (bs, 3H); 7.09-6.70 (m, 3H); 3.79 (s, 3H); 3.72-3.57 (m, 1H); 3.24-2.88 (m, 4H).

Mass: 196 [M+1].

### Example 5

### Resolution of (±)-3,4-dihydro-8-methoxy-1(2H)-benzopyran-3-amine

In absolute ethanol (1200 ml), (±)-3,4-dihydro-8-methoxy-1(2H)-benzopyran-3-amine (26 g; 0.145 moles) and (S)-2-(6-methoxy-2-naphthyl)propionic acid (33.4 g; 0.145 moles) were heated up to complete dissolution.

The solution was brought to room temperature very slowly (about 24 hours).

The solid obtained by filtration was crystallized twice from ethanol giving (S)-2-(6-methoxy-2-naphthyl)propionate of (S)-3,4-dihydro-8-methoxy-1(2H)-benzopyran-3-amine (11.6 g; 19.5% yield).
m.p. 163-164°C
e.e. 97% (HPCE)
[α]_{D}=0° (1%, methanol)

By working in a similar way but using (R)-2-(6-methoxy-2-naphthyl)propionic acid, (R)-2-(6-methoxy-2-naphthyl)propionate of (R)-3,4-dihydro-8-methoxy-1(2H)-benzopyran-3-amine (22.7% yield) was obtained.
m.p. 163-164°C
e.e. 96.2% (HPCE)
[α]_{D}=0° (1%, methanol)

¹H-NMR (200 MHz, CDCl₃): δ (ppm): 7.68-6.52 (m, 9H); 4.13-3.70 (m, 3H); 3.88 (s, 3H); 3.83 (s, 3H); 3.41-3.30 (m, 1H); 3.02-2.49 (m, 2H); 1.51 (d, 3H).

By treatment of the above reported salts with a saturated solution of sodium bicarbonate and extraction with ethyl ether, the following compounds were obtained.

(S)-3,4-dihydro-8-methoxy-1(2H)-benzopyran-3 -amine
hygroscopic white solid,
[α]_{D}=+53^{°} (1%, methanol)

¹H-NMR (200 MHz, CDCl₃): δ (ppm): 6.72 (m, 3H); 4.21 (m, 1H); 3.87 (m, 1H); 3.85 (s, 3H); 3.36 (m, 1H); 3.05 (dd, 1H); 2.56 (dd, 1H); 1.48 (bs, 2H).

(R)-3,4-dihydro-8-methoxy-1(2H)-benzopyran-3-amine hygroscopic white solid,
[α]_{D}=-54.60 (1%, methanol)

### Example 6

### Preparation of (R)-N-[3,4-dihydro-8-methoxy-1(2H)-benzopyran-3-yl]propanamide

Propionyl chloride (3.73 ml; 42.6 mmoles) was added to a solution of (R)-3,4-dihydro-8-methoxy-1(2H)-benzopyran-3-amine (5.6 g; 28.4 mmoles), prepared as described in example 5, and of triethylamine (5.9 ml; 42.6 mmoles) in anhydrous methylene chloride (100 ml), cooled at 0°C and under nitrogen.

The reaction mixture was kept under stirring at room temperature for 2 hours.

After washing with HCl 1% and with a saturated solution of sodium bicarbonate, the organic phase was dried on anhydrous sodium sulphate and evaporated to dryness.

The resultant yellow solid was triturated in ethyl ether giving (R)-N-[3,4-dihydro-8-methoxy-1(2H)-benzopyran-3-yl]propanamide (6.2 g; 93% yield) as a white solid.
m.p. 97-98°C

¹H-NMR (200 MHz, CDCl₃): δ (ppm): 6.87-6.60 (m, 3H); 5.99 (bd, 1H); 4.52-4.41 (m, 1H); 4.29-4.05 (m, 2H); 3.80 (s, 3H); 3.15-2.68 (m, 2H); 2.12 (q, 2H); 1.07 (t, 3H).

By working in a similar way the following compounds were prepared.

N-[3,4-dihydro-7,8-dimethoxy-1(2H)-benzopyran-3-yl]propanamide
white solid (100% yield).
m.p. 106-107°C

¹H-NMR (200 MHz, CDCl₃): δ (ppm): 6.72 (d, 1H); 6.51 (d, 1H); 5.79 (bd, 1H); 4.52-4.40 (m, 1H); 4.29-4.06 (m, 2H); 3.85 (s, 3H); 3.83 (s, 3H); 3.11-2.62 (m, 2H); 2.13 (q, ZH); 1.09 (t, 3H).

N-[3,4-dihydro-7,8-dimethoxy-1(2H)-benzothiopyran-3-yl]propanamide
white solid (97% yield).
m.p. 123-124°C

¹H-NMR (200 MHz, CDCl₃): δ (ppm): 6.73 (d, 1H); 6.61 (d, 1H); 5.91 (bd, 1H); 4.71-4.58 (m, 1H); 3.84-3.81 (2s, 6H); 3.19-2.74 (m, 4H); 2.13 (q, 2H); 1.08 (t, 3H).

Mass: 282 [M+1].

N-[3,4-dihydro-8-methoxy-1(2H)-benzothiopyran-3-yl]propanamide
white solid (98% yield).

¹H-NMR (200 MHz, CDCl₃): δ (ppm): 7.03-6.62 (m, 3H); 5.95 (bd, 1H); 4.73-4.60 (m, 1H); 3.87 (s, 3H); 3.22-2.80 (m, 4H); 2.12 (q, 2H); 1.08 (t, 3H).

Mass: 252 [M+1].

### Example 7

### Preparation of (R)-N-propyl-3,4-dihydro-8-methoxy-1(2H)-benzopyran-3-amine hydrochloride

Borane-dimethylsulphide (6.6 ml; 66.3 mmoles) was added to a solution of (R)-N-[3,4-dihydro-8-methoxy-1(2H)-benzopyran-3-yl]propanamide (5.2 g; 22.1 mmoles), prepared as described in example 6, in anhydrous tetrahydrofuran (100 ml), kept under nitrogen at room temperature.

At the end of the addition, the reaction mixture was heated under reflux for 1 hour.

After cooling to room temperature, a mixture methanol:concentrated HCl=9:1 (180 ml) was added dropwise.

After further heating under reflux for 1 hour, the solvent was evaporated and the residue was collected three times with methanol and twice with ethanol, by evaporating to dryness each time.

(R)-N-propyl-3,4-dihydro-8-methoxy-1(2H)-benzopyran-3-amine hydrochloride (4.4 g; 86% yield) was obtained as a white solid.

m.p. 234-236°C (dec.)

¹H-NMR (200 MHz, CDCl₃): δ (ppm): 10.20-9.70 (bs, 2H); 6.88-6.62 (m, 3H); 4.68-4.40 (m, 2H); 3.81 (s, 3H); 3.73-3.49 (m, 1H); 3.45-2.90 (m, 4H); 2.08-1.87 (m, 2H); 1.01 (t, 3H).

By working in a similar way the following compounds were prepared.

N-propyl-3,4-dihydro-7,8-dimethoxy-1(2H)-benzopyran-3-amine hydrochloride
(89% yield) as a white solid.

m.p. 216-218°C

¹H-NMR (200 MHz, CDCl₃): δ (ppm): 10.10-9.75 (bs, 2H); 6.72 (d, 1H); 6.50 (d, 1H); 4.69-4.33 (m, 2H); 3.81 (s, 6H); 3.70-3.50 (m, 1H); 3.36-2.85 (m, 4H); 2.07-1.88 (m, 2H); 1.01 (t, 3H).

N-propyl-3,4-dihydro-7,8-dimethoxy-1(2H)-benzothiopyran-3-amine hydrochloride
(99% yield) as a white solid.

m.p. 229-231°C (dec.)

¹H-NMR (200 MHz, DMSO-d₆): δ (ppm): 9.30 (m, 2H); 6.88 (d, 1H); 6.79 (d, 1H); 3.78 and 3.69 (2s, 6H); 3.70-3.52 (m, 1H); 3.42-2.89 (m, 6H); 1.79-1.59 (m, 2H); 0.92 (t, 3H).

Mass: 304 [M+1].

N-propyl-3,4-dihydro-8-methoxy-1(2H)-benzothiopyran-3-amine hydrochloride
(98% yield) as a white solid.

¹H-NMR (200 MHz, DMSO-d₆): δ (ppm): 9.38 (m, 2H); 7.09-6.71 (m, 2H); 3.79 (s, 3H); 3.72-3.55 (m, 1H); 3.40-2.90 (m, 6H); 1.79-1.59 (m, 2H); 0.92 (t, 3H).

Mass: 274 [M+1].

### Example 8

### Preparation of 6-[(4-methoxyphenyl)acetylamino]hexanoic acid

N,N-dimethylformamide (0.1 ml) and thionyl chloride (3.5 g; 29.4 mmoles) were added under stirring at room temperature to a solution of 4-methoxyphenylacetic acid (3.3 g; 19.8 mmoles) in methylene chloride (50 ml).

After 1 hour, the solvent was evaporated under reduced pressure obtaining an oil which was dissolved in methylene chloride (6 ml).

The resultant solution was added dropwise, contemporaneously with a solution of sodium hydroxide 4N (5 ml) and under vigorous stirring, to a solution of 6-aminohexanoic acid (2.5 g; 19.1 mmoles) and sodium hydroxide (0.8 g; 20 mmoles) in water (10 ml).

The reaction mixture was kept under stirring at room temperature for 3 hours.

The phases were separated and the aqueous phase was washed with methylene chloride (10 ml), acidified with hydrochloric acid 37% up to pH 1 and extracted with methylene chloride (15 ml).

After drying the resultant organic phase on anhydrous sodium sulphate, the solvent was evaporated under reduced pressure.

The solid residue was triturated in ethyl ether obtaining after filtration 6-[(4-methoxyphenyl)acetylamino]hexanoic acid (4.4 g; 79.5% yield) as a white solid.

m.p. 93-94°C (from ethyl acetate)

¹H-NMR (200 MHz, CDCl₃): δ (ppm): 7.18-6.81 (m, 4H); 5.43 (bt, 1H); 3.79 (s, 3H); 3.49 (s, 2H); 3.17 (m, 2H); 2.28 (t, 2H); 1.66-1.16 (m, 6H).

Mass: 280 [M+1].

By working in a similar way the following compound was prepared.

6-[(3,4-dimethoxyphenyl)acetylamino)hexanoic acid
58% yield

m.p. 75-76°C

¹H-NMR (200 MHz, DMSO-d₆): δ (ppm): 7.94 (bt, 1H); 6.89-6.70 (m, 3H); 3.70 (s, 3H); 3.69 (s, 3H); 3.28 (s, 2H); 2.99 (m, 2H); 2.17 (t, 2H); 1.51-1.12 (m, 6H).

Mass: 310 [M+1].

### Example 9

### Preparation of 6-[(2-methoxy-1,3 -benzothiazol-6-yl)acetylamino]hexanoic acid

Potassium thiocyanate (257 g; 2.64 moles) was added to a solution of 4-aminophenylacetic acid (200 g; 1.32 moles) in water (1000 ml) and concentrated hydrochloric acid (130 g; 1.32 moles), under stirring at 50°C.

The reaction mixture was heated at 100°C for 16 hours, then cooled to 0°C and filtered.

Absolute ethanol (600 ml) was added to the solid and the mixture was heated under reflux for 1 hour.

After cooling to 0°C, filtering and drying the solid under vacuum at 60°C, 4-(2-carboxyethyl)phenylthiourea (173 g) was obtained as a solid.

¹H-NMR (200 MHz, DMSO-d₆): δ (ppm): 12.30 (bs, 1H); 9.64 (s, 1H); 7.70-7.20 (bs, 2H); 7.35-7.16 (m, 4H); 3.52 (s, 2H).

Mass: 211 [M+1].

Bromine (261 g; 1.64 moles) was slowly added to a suspension of 4-(2-carboxyethyl)phenylthiourea (172 g; 0.82 moles) in chloroform (1.7 l), under stirring at 0°C.

After 30 minutes the reaction mixture was heated under reflux for 1 hour, then cooled at 0°C and filtered.

The solid was washed with acetone and dissolved in water (2.5 l); a 10.8N aqueous sodium hydroxide solution (67 ml) was added under stirring at room temperature.

After 1 hour, the solid was filtered and washed with water.

After drying under vacuum at 60°C, 2-amino-6-(2-carboxyethyl)-1,3-benzothiazole (129 g) was obtained.

¹H-NMR (200 MHz, DMSO-d₆): δ (ppm): 12.45-12.11 (bs, 1H); 7.52 (d, 1H); 7.41 (bs, 2H); 7.25 (d, 1H); 7.08 (dd, 1H); 3.55 (s, 2H).

Mass: 209 [M+1].

A solution of sodium nitrite (104 g; 1.5 moles) in water (155 ml) was added dropwise in 2 hours, under stirring at 0°C, to a solution of 2-amino-6-(2-carboxyethyl)-1,3-benzothiazole (104 g; 0.5 moles) in 85% phosphoric acid (1145 ml).

After 1 hour the mixture was added in 1 hour to a solution of CuSO₄·5H₂O (500 g; 2.0 moles) and sodium chloride (584 g; 10.0 moles) in water (2.0 l), under stirring at 0°C.

The temperature of the reaction mixture was let spontaneously arise up to the room value overnight; after filtration, the solid was washed with water and dried under vacuum at 60°C obtaining 2-chloro-6-(2-carboxyethyl)-1,3-benzothiazole (104 g).

¹H-NMR (200 MHz, DMSO-d₆): δ (ppm): 12.42 (bs, 1H); 7.99 (d, 1H); 7.89 (d, 1H); 7.44 (dd, 1H); 3.73 (s, 2H).

Mass: 228 [M+1].

Metallic sodium (17.8 g) was added portionwise to methanol (800 ml) under stirring. After complete dissolution, 2-chloro-6-(2-carboxyethyl)-1,3-benzothiazole (80 g; 0.35 moles) was added and the reaction mixture was heated under reflux for 3 hours.

The solvent was evaporated and water (640 ml), methanol (160 ml) and, under stirring at room temperature, concentrated hydrochloric acid (44 ml) were added to the residue.

After cooling at 0°C and filtering, the solid was dried under vacuum at 60°C obtaining 2-methoxy-6-(2-carboxyethyl)-1,3-benzothiazole (71 g) as a beige solid.

¹H-NMR (200 MHz, DMSO-d₆): δ (ppm): 12.37 (s, 1H); 7.76 (d, 1H); 7.61 (d, 1H); 7.28 (dd, (1H); 4.13 (s, 3H); 3.64 (s, 2H).

Mass: 224 [M+1].

N,N'-dicyclohexylcarbodiimide (1.85 g; 9.0 mmoles) was added to a solution of 2-methoxy-6-(2-carboxyethyl)-1,3-benzothiazole (2.0 g; 9.0 mmoles) and N-hydroxysuccinimide (1.0 g; 9.0 mmoles) in dioxane (15 ml), under stirring at room temperature.

After 1 hour ethyl ether (15 ml) was added and the reaction mixture was filtered.

The solution was evaporated to dryness and the resultant residue was dissolved in dioxane (10 ml) and N,N-dimethylformamide (5 ml).

The resultant solution was added, under stirring at room temperature, to a solution of 6-aminohexanoic acid (1.2 g; 9.0 mmoles) and dicyclohexylamine (1.6 g; 9.0 mmoles) in water (6 ml) and ethanol (6 ml).

After 1 hour, the solvents were evaporated under reduced pressure, water (20 ml) and, under stirring, a solution of potassium hydrogenosulphate (1.3 g; 9.6 mmoles) in water (6 ml) were added.

After 30 minutes the precipitate was filtered and dried under vacuum at 60°C obtaining 6-[(2-methoxy-1,3-benzothiazol-6-yl)acetylamino]hexanoic acid (2.8 g) as a white solid.

¹H-NMR (200 MHz, DMSO-d₆): δ (ppm): 8.03 (t, 1H); 7.71 (d, 1H); 7.58 (d, 1H); 7.26 (dd, 1H); 4.13 (s, 3H); 3.44 (s, 2H); 3.08-2.94 (m, 2H); 2.16 (t, 2H); 1.56-1.12 (m, 6H).

Mass: 337 [M+1].

### Example 10

### Preparation of (R)-N-propyl-N-[6-[(4-methoxyphenyl)acetylammo]-1-oxohexyl]-3,4-dihydro-8-methoxy-3-[1(2H)-benzopyranyl]amine

Thionyl chloride (3.57 g; 30 mmoles) was added to a suspension of 6-[(4-methoxyphenyl)acetylamino]hexanoic acid (5.6 g; 20 mmoles), prepared as described in example 8, in anhydrous methylene chloride (60 ml), under nitrogen at room temperature.

The solution was kept for 1 hour at room temperature and the solvent was evaporated under reduced pressure obtaining a thick oil (2.53 g) which was dissolved in anhydrous methylene chloride (10 ml).

The resultant solution was added dropwise to a solution obtained by adding triethylamine (1.77 g; 17.5 mmoles), under stirring at room temperature, to a suspension of (R)-N-propyl-3,4-dihydro-8-methoxy-1(2H)-benzopyran-3-amine hydrochloride (1.5 g; 5.82 mmoles), prepared as described in example 7, in anhydrous methylene chloride (50 ml).

The reaction mixture was kept under stirring at room temperature for 2 hours.

After washing with HCl 1% and with a saturated solution of sodium bicarbonate, the organic phase was dried on anhydrous sodium sulphate and evaporated to dryness under reduced pressure obtaining (R) -N-propyl-N-[6-[(4-methoxyphenyl)acetylamino]-1-oxohexyl]-3,4-dihydro-8-methoxy-3-[1(2H)-benzopyranyl]amine (2.8 g; 100% yield) as a yellow solid.

¹H-NMR (200 MHz, CDCl₃): δ (ppm): 7.18-6.61 (m, 7H); 5.60-5.40 (in, 1H); 4.40-3.88 (m, 3H); 3.86 (s, 3H); 3.78 (s, 3H); 3.48 (s, 2H); 3.33-2.79 (m, 6H); 2.43-2.22 (m, 2H); 1.69-1.20 (m, 8H); 0.85 (t, 3H).

Mass: 483 [M+1].

By working in a similar way the following compounds were prepared:

(R)-N-propyl-N-[6-[(3,4-dimethoxyphenyl)acetylamino]-1-oxohexyl]-3,4-dihydro-8-methoxy-3-[1(2H)-benzopyranyl]amine (94.2% yield) as a thick oil.

¹H-NMR (200 MHz, CDCl₃): δ (ppm): 6.90-6.61 (m, 6H); 5.67-5.51 (m, 1H); 4.45-3.90 (m, 3H); 3.85 (s, 9H); 3.49 (s, 2H); 3.33-2.80 (m, 6H); 2.39-2.22 (m, 2H); 2.15-1.20 (m, 8H); 0.85 (t, 3H).

Mass: 513 [M+1].

N-propyl-N-[6-[(3,4-dimethoxyphenyl)acetylamino]-1-oxohexyl]-3,4-dihydro-7,8-dimethoxy-3-[1(2H)-benzopyranyl]amine (98.3% yield) as a thick oil.

¹H-NMR (200 MHz, CDCl₃): δ (ppm): 6.85-6.44 (m, 5H); 5.62-5.50 (m, 1H); 4.49-3.92 (m, 3H); 3.86 and 3.83 and 3.81 (3s, 12H); 3.49 (s, 2H); 3.27-2.64 (m, 6H); 2.39-2.23 (m, 2H); 1.70-1.20 (m, 8H); 0.85 (t, 3H).

Mass: 543 [M+1].

N-propyl-N-[6-[(4-methoxyphenyl)acetylamino]-1-oxohexyl]-3,4-dihydro-7,8-dimethoxy-3-[1(2H)-benzopyranyl]amine (100% yield) as a waxy solid.

¹H-NMR (200 MHz, CDCl₃): δ (ppm): 7.15-6.45 (m, 6H); 5.60-5.48 (m, 1H); 4.49-3,89 (m, 3H); 3.83 and 3.81 and 3.78 (3s, 9H); 3.48 (s, 2H); 3.25-2.75 (m, 6H); 2.42-2.22 (m, 2H); 1.68-1.20 (m, 8H); 0.85 (t, 3H).

Mass: 513 [M+1].

N-propyl-N-[6-[(2-methoxy-6-benzothiazolyl)acetylamino]-1-oxohexyl]-3,4-di-hydro-7,8-dimethoxy-3-[1(2H)-benzopyranyl]amine (69% yield) as a thick oil.

¹H-NMR (200 MHz, CDCl₃): δ (ppm): 7.61-7.15 (m, 3H); 6.76-6.42 (m, 2H); 5.90-5.79 (m, 1H); 4.43-3.85 (m, 3H); 4.15 (s, 3H); 3.82 and 3.78 (2s, 6H); 3.55 (s, 2H); 3.25-2.73 (m, 6H); 2.36-2.20 (m, 2H); 1.65-1.20 (m, 8H); 0.83 (t, 3H).

Mass: 570 [M+1].

(R)-N-propyl-N-[6-[(2-methoxy-6-benzothiazolyl)acetylamino]-1-oxohexyl]-3,4-dihydro-8-methoxy-3-[1(2-benzopyranyl]amine (54% yield) as a sticky yellow oil.

¹H-NMR (200 MHz, CDCl₃): δ (ppm): 7.64-7.18 (m, 3H); 6.90-6.60 (m, 3H); 5.71-5.63 (m, 1H); 4.40-3.91 (m, 3H); 4.18 (s, 3H); 3.86 and 3.84 (2s, 3H); 3.59 (s, 2H); 3.34-2.63 (m, 6H); 2.38-2.22 (m, 2H); 1.70-1.22 (m, 8H); 0.94-0.80 (m, 3H).

Mass: 540 [M+1].

N-propyl-N-[6-[(3,4-dimethoxyphenyl)acetylamino]-1-oxohexyl]-3,4-dihydro-8-methoxy-3-[1(2H)-benzothiopyranyl]amine (94% yield) as a yellow amorphous solid.

¹H-NMR (200 MHz, CDCl₃): δ (ppm): 7.05-6.61 (m, 6H); 5.68-5.55 (m, 1H); 4.50-4.19 (m, 1H); 3.84 (m, 9H); 3.48 (s, 2H); 3.42-2.77 (m, 8H); 2.34-2.24 (m, 2H); 1.68-1.24 (m, 8H); 0.95-0.82 (m, 3H).

Mass: 529 [M+1].

N-propyl-N-[6-[(4-methoxyphenyl)acetylammo]-1-oxohexyl]-3,4-dihydro-8-methoxy-3-[1(2H)-benzothiopyranyl]amine (100% yield) as a yellow oil.

¹H-NMR (200 MHz, CDCl₃): δ (ppm): 7.18-6.81 (m, 4H); 7.05-6.61 (m, 3H); 5.59-5.49 (m, 1H); 4.50-4.19 (m, 1H); 3.85-3.76 (m, 6H); 3.48-2.76 (m, 10H); 2.35-2.23 (m, 2H); 1.70-1.22 (m, 8H); 0.95-0.84 (m, 3H).

Mass: 499 [M+1].

N-propyl-N-[6-[(2-methoxy-6-benzothiazolyl)acetylamino]-1-oxohexyl]-3,4-dihydro-8-methoxy-3-[1(2H)-benzothiopyranyl]amine (64% yield) as a white solid.

¹H-NMR (200 MHz, CDCl₃): δ (ppm): 7.62-7.18 (m, 3H); 7.04-6.59 (m, 3H); 6.90-6.77 (m, 1H); 4.51-4.22 (m, 1H); 4.15 (s, 3H); 3.74 and 3.71 (2s, 3H); 3.58 (s, 2H); 3.48-2.74 (m, 8H); 2.33-2.20 (m, 2H); 1.68-1.18 (m, 8H); 0.93-0.82 (m, 3H).

Mass: 556 [M+1].

N-propyl-N-[6-[(2-methoxy-6-benzothiazolyl)acetylamino]-1-oxohexyl]-3,4-dihydro-7,8-dimethoxy-3-[1(2H)-benzothiopyranyl]amine (100% yield) as an orange oil.

¹H-NMR (200 MHz, CDCl₃): δ (ppm): 7.64-7.18 (m, 3H); 6.77-6.55 (m, 2H); 5.76-5.55 (m, 1H); 4.47-4.21 (m, 1H); 4.17 (s, 3H); 3.82-3.79 (m, 6H); 3.58 (s, 2H); 3.43-2.68 (m, 8H); 2.41-2.22 (m, 2H); 1.67-1.19 (m, 8H); 0.95-0.83 (m, 3H).
Mass: 586 [M+1].

N-propyl-N-[6-f(3,4-dimethoxyphenyl)acetylamino]-1-oxohexyl]-3,4-dihydro-7,8-dimethoxy-3-[1(2H)-benzothiopyranyl]amine (86% yield) as a yellow oil.

¹H-NMR (200 MHz, CDCl₃): δ (ppm): 6.83-6.55 (m, 5H); 5.66-5.56 (m, 1H); 4.49-4.16 (m, 1H); 3.84-3.79 (m, 12H); 3.47 (s, 2H); 3.43-2.69 (m, 8H); 2.34-2.23 (m, 2H); 1.67-1.20 (m, 8H); 0.93-0.83 (m, 3H).

Mass: 559 [M+1].

N-propyl-N-[6-[(4-methoxyphenyl)acetylamino]-1-oxohexyl]-3,4-dihydro-7,8-dimethoxy-3-[1(2H)-benzothiopyranyl]amine (94% yield) as a yellow oil.

¹H-NMR (200 MHz, CDCl₃): δ (ppm): 7.18-6.82 (m, 4H); 6.81-6.55 (m, 2H); 5.63-5.53 (m, 1H); 4.50-4.18 (m, 1H); 3.83-3.76 (m, 9H); 3.48 (s, 2H); 3.47-2.69 (m, 8H); 2.36-2.23 (m, 2H); 1.70-1.20 (m, 8H); 0.95-0.82 (m, 3H).

Mass: 529 [M+1].

### Example 11

### Preparation of N-propyl-N-[6-[(2,3-dihydro-2-oxo-6-benzothiazolyl)acetylamino]-1-oxohexyl]-3,4-dihydro-8-methoxy-3-[1(2H)-benzothiopyranyl]amine

Concentrated hydrochloric acid (37%; 0.3 ml; 0.0036 moles) was slowly added to a stirred solution of N-propyl-N-[6-[(2-methoxy-6-benzothiazolyl)acetylamino]-1-oxohexyl]-3,4-dihydro-8-methoxy-3-[1(2H)-benzothiopyranyl]amine (1 g; 0.0018 moles), prepared as described in example 10, in dioxane (10 ml).

The resulting mixture was stirred for 1.5 hours, at room temperature, concentrated under reduced pressure, taken up with tetrahydrofuran (50 ml) and washed with aqueous sodium bicarbonate.

The organic layer was dried on anhydrous sodium sulphate and evaporated to afford N-propyl-N-[6-[(2,3-dihydro-2-oxo-6-benzothiazolyl)acetylamino)-1-oxohexyl]-3,4-dihydro-8-methoxy-3-[1(2H)-benzothiopyranyl]amine (1 g; 100% yield) as a white foam.

¹H-NMR (200 MHz, CDCl₃): δ (ppm): 7.21-6.60 (m, 6H); 6.31-6.15 (m, 1H); 4.56-4.19 (m, 1H); 3.85 and 3.82 (2s, 3H); 3.49 (s, 2H); 3.48-2.76 (in, 8H); 2.38-2.25 (m, 2H); 1.70-1.20 (m, 8H); 0.95-0.82 (m, 3H).

Mass: 542 [M+1].

By working in a similar way the following compounds were prepared.

N-propyl-N-[6-[(2,3-dihydro-2-oxo-6-benzothiazolyl)acetylamino)-1-oxohexyl]-3,4-dihydro-7,8-dimethoxy-3-[1(2H)-benzothiopyranyl]amine (77% yield) as a brown foam.

¹H-NMR (200 MHz, CDCl₃, 60°C): δ (ppm): 7.08-6.53 (m, 5H); 6.07 (bs, 1H); 4.43-4.13 (m, 1H); 3.83-3.75 (m, 6H); 3.39 (bs, 2H); 3.31-2.69 (in, 8H); 2.27-2.20 (m, 2H); 1.65-1.17 (m, 8H); 0.92-0.82 (m, 3H).

Mass: 572 [M+1].

N-propyl-N-[6-[(2.3-dihydro-2-oxo-6-benzothiazolyl)acetylamino]-1-oxohexyl]-3,4-dihydro-7,8-dimethoxy-3-[1(2H)-benzopyranyl]amine (98% yield) as a white solid.

¹H-NMR (200 MHz, CDCl₃): δ (ppm): 7.11-6.47 (m, 5H); 6.10-5.98 (m, 1H); 4.53-3.90 (m, 3H); 3.85 and 3.81 (2s, 6H); 3.52 (s, 2H); 3.30-2.78 (m, 6H); 2.39-2.28 (m, 2H); 2.00-1.20 (m, 8H); 0.86 (t, 3H).

Mass: 556 [M+1].

(R)-N-propyl-N-[6-[(2,3-dihydro-2-oxo-6-benzothiazolyl)acetylamino]-1-oxohexyl] -3,4-dihydro-8-methoxy-3-[1(2H)-benzopyranyl]amine (100% yield) as a white solid.

¹H-NMR (200 MHz, CDCl₃): δ (ppm): 7.25-6.63 (m, 6H); 6.03-5.91 (m, 1H); 4.57-4.13 (m, 3H); 3.87 (s, 3H); 3.52 (s, 2H); 3.31-2.82 (m, 6H); 2.39-2.28 (m, 2H); 1.92-1.20 (m, 8H); 0.86 (t, 3H).

Mass: 526 [M+1].

### Example 12

### Preparation of (R)-N-propyl-N-[6-[2-(4-methoxyphenyl)ethylamino]hexyl]-3,4-dihydro-8-methoxy-3-[1(2H)-benzopyranyl]amine dihydrochloride

By working in a way similar to that described in example 7 but using (R)-N-propyl-N-[6-[(4-methoxyphenyl)acetylamino]-1-oxohexyl]-3,4-dihydro-8-methoxy-3-[1(2H)-benzopyranyl]amine (3.36 g; 6.99 mmoles), prepared as described in example 10, anhydrous tetrahydrofuran (100 ml) and borane-dimethylsulphide (4.5 ml; 45.5 mmoles), (R)-N-propyl-N-[6-[2-(4-methoxyphenyl)ethylamino]hexyl]-3,4-dihydro-8-methoxy-3-[1(2H)-benzopyranyl]amine dihydrochloride (3.63 g; 98% yield) was obtained as an amorphous solid.

¹H-NMR (200 MHz, D₂O): δ (ppm): 7.15-6.67 (m, 7H); 4.45-4.19 (m, 2H); 3.90-3.82 (m, 1H); 3.69 (s, 3H); 3.66 (s, 3H); 3.51-2.77 (m, 12H); 1.68-1.15 (m, 10H); 0.80 (t, 3H).

Mass: 455 [M+1].

Analogously the following compounds were prepared.

(R)-N-propyl-N-[6-[2-(3,4-dimethoxyphenyl)ethylamino]hexyl]-3,4-dihydro-8-methoxy-3-[1(2H)-benzopyranyl]amine dihydrochloride (94% yield) as a white amorphous solid.

¹H-NMR (200 MHz, DMSO-d₆): δ (ppm): 11.0 (bs, 1H); 9.09 (bs, 2H); 6.90-6.70 (m, 6H); 4.51-4.23 (m, 2H); 3.91-3.78 (m, 1H); 3.75 and 3.73 and 3.71 (3s, 9H); 3.70-2.80 (m, 12H); 1.85-1.25 (m, 10H); 0.90 (t, 3H).

Mass: 558 [M+1].

N-propyl-N-[6-[2-(4-methoxyphenyl)ethylamino]hexyl]-3,4-dihydro-7,8-dimethoxy-3-[1(2H)-benzopyranyl]amine dihydrochloride (100% yield) as an amorphous solid.

¹H-NMR (200 MHz, D₂O): δ (ppm): 7.12-6.58 (m, 6H); 4.48-4.20 (m, 2H); 3.90-3.80 (m, 1H); 3.67 and 3.65 and 3.62 (3s, 9H); 3.25-2.72 (m, 12H); 1.67-1.12 (m, 10H); 0.79 (t, 3H).

Mass: 485 [M+1].

N-propyl-N-[6-[2-(3,4-dimethoxyphenyl)ethylamino]hexyl]-3,4-dihydro-7,8-dimethoxy-3-[1(2H)-benzopyranyl]amine dihydrochloride (95% yield) as an amorphous solid.

¹H-NMR (200 MHz, CDCl₃): δ (ppm): 6.71-6.42 (m, 5H); 4.40-3.82 (m, 2H); 3.85 and 3.84 and 3.82 and 3.81 (4s, 12H); 3.19-3.02 (m, 1H); 2.88-2.42 (m, 12H); 1.60-1.22 (m, 10H); 0.84 (t, 3H).

Mass: 515 [M+1].

N-propyl-N-[6-[2-(4-methoxyphenyl)ethylamino]hexyl]-3,4-dihydro-8-methoxy-3-[1(2H)-benzothiopyranyl]amine dihydrochloride (100% yield) as a white foam.

¹H-NMR (200 MHz, CDCl₃): δ (ppm): 11.40 (bs, 1H); 9.70 (bs, 2H); 7.20-6.63 (m, 7H); 4.05-3.87 (m, 1H); 3.82 and 3.75 (2s, 6H); 3.50-2.85 (m, 14H); 2.05-1.40 (m, 10H); 1.01 (bt, 3H).

Mass: 471 [M+1].

N-propyl-N-[6-[2-(3,4-dimethoxyphenyl)ethylamino]hexyl]-3,4-dihydro-8-methoxy-3-[1(2H)-benzothiopyranyl]amine dihydrochloride (90% yield) as an amorphous solid.

¹H-NMR (200 MHz, DMSO-d₆): δ (ppm): 10.98 (bs, 1H); 9.15 (bs, 2H); 7.10-6.71 (m, 6H); 3.90-3.70 (m, 1H); 3.80 and 3.74 and 3.71 (3s, 9H); 3.60-2.70 (m, 14H); 1.90-1.25 (m, 10H); 0.91 (t, 3H).

Mass: 501 [M+1].

N-propyl-N-[6-[2-(2,3-dihydro-2-oxo-6-benzothiazolyl)ethylamino]hexyl]-3,4-dihydro-8-methoxy-3-[1(2H)-benzothiopyranyl]amine dihydrochloride (98% yield) as a white solid.

¹H-NMR (200 MHz, DMSO-d₆): δ (ppm): 11.99 (s, 1H); 11.03 (bs, 1H); 9.20 (bs, 2H); 7.47-7.06 (m, 3H); 6.88 (d, 1H); 6.80 (d, 1H); 4.05-3.83 (m, 1H); 3.76 and 3.68 (2s, 6H); 3.49-2.78 (m, 14H); 1.85-1.28 (m, 10H); 0.98-0.84 (m, 3H).

Mass: 514 [M+1].

N-propyl-N-[6-f2-(3,4-dimethoxyphenyl)ethylamino]hexyl]-3 4-dihydro-7,8-dimethoxy-3-[1(2H)-benzothiopyranyl]amine dihydrochloride (100% yield) as a white solid.

¹H-NMR (200 MHz, CDCl₃): δ (ppm): 11.3 (bs, 1H); 9.67 (bs, 2H); 6.88-6.59 (m, 5H); 3.97-3.84 (m, 1H); 3.82 and 3.81 and 3.79 and 3.78 (4s, 12H); 2.45-2.88 (m, 14H); 2.05-1.46 (m, 10H); 1.04-0.96 (m, 3H).

Mass: 531 [M+1].

N-propyl-N-[6-[2-(4-methoxyphenyl)ethylamino]hexyl]-3,4-dihydro-7,8-dimethoxy-3-[1(2H)-benzothiopyranyl]amine dihydrochloride (95% yield) as a white amorphous solid.

¹H-NMR (200 MHz, CDCl₃): δ (ppm): 11.3 (bs, 1H); 9.60 (bs, 2H); 7.64-7.15 (m, 4H); 6.87-6.58 (m, 2H); 3.93-3.77 (m, 1H); 3.78 and 3.76 and 3.72 (3s, 9H); 2.86-2.46 (m, 14H); 1.99-1.39 (m, 10H); 1.02-0.95 (m, 3H).

Mass: 501 [M+1].

N-propyl-N-[6-[2-(2,3-dihydro-2-oxo-6-benzothiazolyl)ethylamino]hexyl]-3,4-dihydro-7,8-dimethoxy-3-[1(2H)-benzothiopyranyl]amine dihydrochloride (100% yield) as a white solid.

¹H-NMR (200 MHz, DMSO-d₆): δ (ppm): 11.99 (s, 1H); 11.03 (bs, 1H); 9.20 (bs, 2H); 7.47-7.06 (m, 3H); 6.88 (d,1H); 6.80 (d, 1H); 4.05-3 .83 (m, 1H); 3.76 and 3.68 (2s, 6H); 3.49-2.78 (m, 14H); 1.85-1.28 (m, 10H); 0.98-0.84 (m, 3H).

Mass: 544 [M+1].

N-propyl-N-[6-[2-(2,3-dihydro-2-oxo-6-benzothiazolyl)ethylamino]hexyl]-3,4-dihydro-7,8-dimethoxy-3-[1(2H)-benzopyranyl]amine dihydrochloride (100% yield) as a white solid.

¹H-NMR (200 MHz, D₂O): δ (ppm): 7.29-7.00 (m, 3H); 6.78 (d,1H); 6.58 (d, 1H); 4.46-4.18 (m, 2H); 3.88-3.78 (m, 1H); 3.65 and 3.60 (2s, 6H); 3.22-2.78 (m, 12H); 1.67-1.10 (m, 10H); 0.77 (t, 3H).

Mass: 528 [M+1].

N-propyl-N-[6-[2-(2,3-dihydro-2-oxo-6-benzothiazolyl)ethylamino]hexyl]-3,4-dihydro-8-methoxy-3-[1(2H)-benzopyranyl]amine dihydrochloride (100% yield) as a white solid.

¹H-NMR (200 MHz, D₂O): δ (ppm): 7.22-6.61 (m, 6H); 4.39-4.11 (m, 2H); 3.86-3.76 (m, 1H); 3.64 (s, 3H); 3.24-2.80 (m, 12H); 1.65-1.12 (m, 10H); 0.77 (t, 3H).

Mass: 498 [M+1].

### Example 13

### Preparation of (R)-N-propyl-N-[6-[2-(4-hydroxyphenyl)ethylamino]hexyl]-3,4-dihydro-8-hydroxy-3-[1(2H)-benzopyranyl]amine dihydrobromide (Compound 1)

A solution of (R)-N-propyl-N-[6-[2-(4-methoxyphenyl)ethylamino]hexyl]-3,4-dihydro-8-methoxy-3-[1(2H)-benzopyranyl]amine dihydrochloride (3.6 g; 6.9 mmoles), prepared as described in example 12, in hydrobromic acid 48% (25 ml) was kept under reflux for 3 hours under nitrogen.

The mixture was concentrated under reduced pressure, collecting several times with absolute ethanol and then with ethanol saturated with HCl.

After evaporation to dryness, the crude (3.94 g) was purified by chromatography (eluent (CH₂Cl₂:CH₃OH:HCOOH:H₂O=80:20:2:2) obtaining Compound 1 (1.89 g; 47% yield) as an amorphous beige solid.

¹H-NMR (200 MHz, D₂O): δ (ppm): 7.07-6.60 (m, 7H); 4.48-4.20 (m, 2H); 3.92-3.83 (m, 1H); 3.31-2.74 (m, 12H); 1.69-1.14 (m, 10H); 0.80 (t, 3H).

Mass: 427 [M+1].

By working in a similar way the following compounds were prepared.

(R)-N-propyl-N-[6-[2-(3,4-dihydroxyphenyl)ethylamino]hexyl]-3,4-dihydro-8-hydroxy-3-[1(2H)-benzopyranyl]amine dihydrobromide (Compound 2) as a brown amorphous solid (73% yield)

¹H-NMR (200 MHz, D₂O): δ (ppm): 6.79-6.56 (m, 6H); 4.48-4.20 (m, 2H); 3.92-3.83 (in, 1H); 3.31-2.70 (m, 12H); 1.70-1.14 (m, 10H); 0.80 (t, 3H).

Mass: 443 [M+1].

N-propyl-N-[6-[2-(3,4-dihydroxyphenyl)ethylamino]hexyl]-3,4-dihydro-7,8-dihydroxy-3-[1(2H)-benzopyranyllamine dihydrobromide (Compound 3) as a beige amorphous solid (70% yield)

¹H-NMR (200 MHz, D₂O): δ (ppm): 6.74 (d, 1H); 6.68 (d, 1H); 6.59 (dd, 1H); 6.50 (d, 1H); 6.43 (d, 1H); 4.47-4.17 (m, 2H); 3.87-3.78 (m, 1H); 3.22-2.69 (in, 12H); 1.68-1.11 (m, 10H); 0.80 (t, 3H).

Mass: 459 [M+1].

N-propyl-N-[6-[2-(3,4-dihydroxyphenyl)ethylamino]hexyl]-3,4-dihydro-8-hydroxy-3-[1(2H)-benzothiopyranyl]amine dihydrobromide (Compound 4) as a light brown solid (84% yield)

¹H-NMR (200 MHz, D₂O): δ (ppm): 6.92-6.53 (m, 6H); 3.98-3.86 (m, 1H); 3.14-3.01 (m, 10H); 2.87-2.79 (m, 2H); 2.74-2.67 (m, 2H); 1.68-1.19 (m, 10H); 0.79 (t, 3H).

Mass: 459 [M+1].

N-propyl-N-[6-[2-(4-hydroxyphenyl)ethylamino]hexyl]-3,4-dihydro-8-hydroxy-3-[1(2H)-benzothiopyranyl]amine dihydrobromide (Compound 5) as a white solid (33% yield)

¹H-NMR (200 MHz, D₂O): δ (ppm): 7.05-6.66 (m, 4H); 6.92-6.60 (m, 3H); 3.96-3.83 (m, 1H); 3.12-2.71 (m, 14H); 1.68-1.21 (m, 10H); 0.80 (t, 3H).

Mass: 443 [M+1].

N-propyl-N-[6-[2-(2,3-dihydro-2-oxo-6-benzothiazolyl)ethylamino]hexyl]-3,4-dihydro-7,8-dihydroxy-3-[1(2H)-benzothiopyranyl]amine dihydrochloride (Compound 6) as a white foam (49% yield).

¹H-NMR (200 MHz, D₂O): δ (ppm): 7.26-6.98 (m, 3H); 6.56-6.47 (m, 2H); 3.92-3.79 (m, 1H); 3.17-2.81 (m, 14H); 1.64-1.16 (m, 10H); 0.78 (t, 3H).

Mass: 515 [M+1].

N-propyl-N-[6-[2-(2,3-dihydro-2-oxo-6-benzothiazolyl)ethylamino]hexyl-3,4-dihydro-8-hydroxy-3-[1(2H)-benzothiopyranyl]amine dihydrochloride (Compound 7) as a white solid (30% yield).

¹H-NMR (200 MHz, DMSO-d₆): δ (ppm): 11.89 (bs, 1H); 10.28 (bs, 1H); 9.98 (bs, 1H); 8.75 (bs, 2H); 7.47-6.59 (m, 6H); 3.95-3.70 (bm, 1H); 3.37-2.83 (m, 14H); 1.71-1.33 (m, 10H); 0.91 (t, 3H).

Mass: 500 [M+1].

N-propyl-N-[6-[2-(3,4-dihydroxyphenyl)ethylamino]hexyl]-3,4-dihydro-7,8-dihydroxy-3-[1(2H)-benzothiopyranyl]amine dihydrobromide (Compound 8) as an amorphous solid (45% yield)

¹H-NMR (200 MHz, D₂O): δ (ppm): 6.73-6.54 (m, 5H); 3.96-3.83 (m, 1H); 3.13-2.67 (m, 14H); 1.68-1.15 (m, 10H); 0.79 (t, 3H).

Mass: 475 [M+1].

N-propyl-N-[6-[2-(4-hydroxyphenyl)ethylamino]hexyl]-3,4-dihydro-7,8-dihydroxy-3-[1(2H)-benzothiopyranyl]amine dihydrobromide (Compound 9) as an amorphous solid (42% yield)

¹H-NMR (200 MHz, D₂O): δ (ppm): 7.05-6.69 (m, 4H); 6.59-6.51 (m, 2H); 3.96-3.83 (m, 1H); 3.20-2.73 (m, 14H); 1.68-1.19 (m, 10H); 0.79 (t, 3H).

Mass: 459 [M+1].

N-propyl-N-[6-[2-(4-hydroxyphenyl)ethylamino]hexyl]-3,4-dihydro-7,8-dihydroxy-3-[1(2H)-benzopyranyl]amine dihydrobromide (Compound 10) as a light brown solid (68% yield)

¹H-NMR (200 MHz, D₂O): δ (ppm): 7.03-6.66 (m, 4H); 6.46 (d, 1H); 6.38 (d, 1H); 4.44-4.13 (m, 2H); 3.83-3.75 (m, 1H); 3.18-2.70 (m, 12H); 1.63-1.08 (m, 10H); 0.76 (t, 3H).

Mass: 443 [M+1].

N-propyl-N-[6-[2-(2,3-dihydro-2-oxo-6-benzothiazolyl)ethylamino]hexyl]-3,4-dihydro-7,8-dihydroxy-3-[1(2H)-benzopyranyl]amine dihydrobromide (Compound 11) as a light brown solid (66% yield).

¹H-NMR (200 MHz, D₂O): δ (ppm): 7.23-6.93 (m, 3H); 6.43 (d, 1H); 6.36 (d, 1H); 4.39-4.13 (m, 2H); 3.80-3.72 (m, 1H); 3.17-2.80 (m, 12H); 1.63-1.12 (m, 10H); 0.81-0.74 (m, 3H).

Mass: 500 [M+1].

(R)-N-propyl-N-[6-[2-(2,3-dihydro-2-oxo-6-benzothiazolyl)ethylamino]hexyl]-3,4-dihydro-8-hydroxy-3-[1(2H)-benzopyranyl]amine dihydrobromide (Compound 12) as a light brown solid (45% yield).

¹H-NMR (200 MHz, DMSO-d₆): δ (ppm): 11.90 (bs, 1H); 9.60 (bs, 1H); 9,14 (bs, 1H); 8.54 (bs, 2H); 7.48 (d, 1H); 7.17 (dd, 1H); 7.08 (d, 1H); 6.77-6.55 (m, 3H); 4.52-4.24 (m, 2H); 3.96-3.86 (m, 1H); 3.25-2.85 (m, 12H); 1.78-1.32 (m, 10H); 0.91 (t,3H).

Mass: 484 [M+1].

### Example 14

### Preparation of N-propyl-N-[6-[2-(4-methoxyphenyl)ethylamino]hexyl]-3,4-dihydro-7,8-dihydroxy-3-[1(2H)-benzopyranyl]amine dihydrochloride (Compound 13)

Ethanthiol (3.3 ml; 45 mmoles) was added to a suspension of sodium hydride (1.12 g; 80% in oil; 37.5 mmoles) in anhydrous N, N-dimethylformamide (10 ml), cooled at 0°C and under nitrogen, and the mixture was kept under stirring at room temperature for 15 minutes.

After addition of N-propyl-N-[2-(4-methoxyphenyl)ethylamino]hexyl]-3,4-dihydro-7,8-dimethoxy-3-[1(2H)-benzopyranyl]amine dihydrochloride (1.7 g; 3 mmoles), prepared as described in example 12, the reaction mixture was kept under reflux for 5 hours.

After evaporation of the solvent to dryness, the residue was collected with water and extracted with ethyl ether.

The aqueous phase was acidified and extracted again with ethyl acetate.

The organic phase was dried on anhydrous sodium sulphate and concentrated under vacuum.

The resultant crude residue (0.6 g) was purified by flash chromatography (eluent CH₂Cl₂:CH₃OH:HCOOH:H₂O=80:20:2:2) obtaining Compound 13 (0.3 g; 19% yield) as a nut-brown solid.

m.p. 103°C (dec.)

¹H-NMR (200 MHz, D₂O): δ (ppm): 7.07-6.71 (m, 4H); 6.59 (s, 2H); 4.48-4.18 (m, 2H); 3.88-3.79 (m, 1H); 3.68 (s, 3H); 3.25-2.74 (m, 12H); 1.65-1.14 (m, 10H); 0.80 (t, 3H).

Mass: 457 [M+1].

### Example 15

### Dopaminergic functional studies on isolated tissues

### Evaluation of D₁-like activity in the Rabbit Splenic Artery (RSA) test

Arterial rings were prepared according to Semeraro et al., Naunyn. Schmied. Arch. Pharmacol., 1990, 342, 539.

The arterial preparations were contracted with U46619 (9,11-dideoxy-11α,9α-epoxy-methanoprostaglandin F_{2α}) at the submaximal concentration of 0.1 µM.

The tested compounds were cumulatively administered.

Dopamine and 7,8-dihydroxy-3,4-dihydro-3-(di-n.propylamino)-1(2H)-benzopyran (Ref. 1), described in Eur. J. Med. Chem. (1991), 26, 497-504, were used as reference substances.

The agonist activity expressed as pD2 is reported in table 1.

### Evaluation of D₂-like activity in the Rabbit Ear Artery (REA) test

Arterial rings were prepared according to the method described by Steinsland et al., Science, 1978, 443, 199, modified as follows.

Male New Zealand rabbits weighing 2.5-3 Kg were sacrificed by intravenous injection of an excess of pentobarbital sodium and exsanguinated. The two ears were taken away and the central ear artery was dissected into 3 mm long rings.

These preparations were set up in a 25 ml organ bath containing Krebs solution (mM/l): NaCl 118, KCl 4.7, CaCl₂ 2.5, MgSO₄ 1.2, NaHCO₃ 25, KH₂PO₄ 1.2, glucose 11.1, equilibrated with 95% O₂-5% CO₂ and maintained at 35±1°C.

Krebs solution was medicated with EDTA (10 µM) to prevent cathecolamine oxidation desipramine (0.1µM) and corticosterone (30 µM), to block neuronal and extraneuronal cathecolamine uptake.

The preparations were electrically stimulated (10 Hz, 1 msec, 40-80 mA, 500 msec duration) at 5 minutes intervals.

The tested compounds were cumulatively administered.

Dopamine and Ref. 1 were used as reference substances.

The agonist activity expressed as pD2 is reported in table 1.

**Table 1**

| | | |
|---|---|---|
| D₁-like and D₂-like activity of compounds 3, 6, 8-11, dopamine and Ref. 1 determined by RSA and REA tests respectively, expressed as pD2. | | |

| | D₁-like (RSA) pD2 | D₂-like (REA) pD2 |
|---|---|---|
| Compound 3 | 7.0 | 8.7 |
| Compound 6 | 7.0 | 8.7 |
| Compound 8 | 7.0 | 10.6 |
| Compound 9 | 7.1 | 9.2 |
| Compound 10 | 6.3 | 9.8 |
| Compound 11 | 7.4 | 9.1 |
| Dopamine | 6.4 | 7.8 |
| Ref. 1 | 6.3 | 9.2 |

The above data show that the compounds of formula I, object of the present invention, have a significant dopaminergic activity.

This activity is higher than that of the reference substances.

## Claims

1. A compound of formula 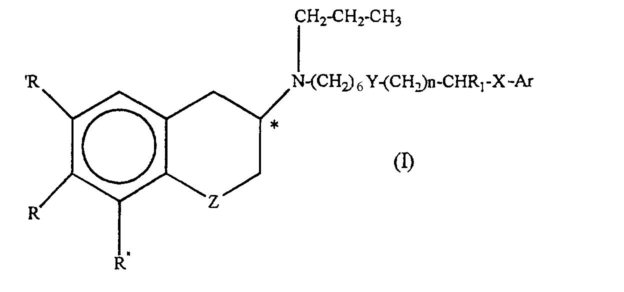 wherein
Z is O, S, SO or SO₂;
R, R' and R" are hydrogen atoms or OH groups, provided that at least one among R, R' and R" is a hydrogen atom but R, R' and R" are not all contemporaneously hydrogen atoms and R' and R" are not both contemporaneously OH groups;
n is an integer number selected among 0, 1, 2, 3 and 4;
R₁ is a hydrogen atom or a C₁-C₄ alkyl group;
Ar is a phenyl of formula 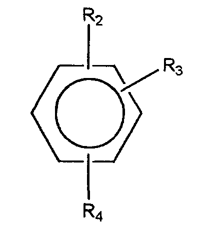 wherein
R₂, R₃ and R₄, the same or different, are hydrogen, OH, halogen, C₁-C₄ alkyl, C₁-C₄ alkoxy, nitro, C₁-C₄ alkylthio, NH₂, mono- or di-C₁-C₄ alkylamino, SH, C₁-C₄ alkylsulphonyl, C₁-C₄ alkoxycarbonyl, NHCHO, C₁-C₄ alkylcarbonylamino, NHCONH₂, C₁-C₄ alkylsulphonylamino, C₁-C₄ alkylaminosulphonyl, SO₂NH₂, NHSO₂NH₂, COOH, SO₃H, CONH₂, CH₂OH or phenyl; or
R₂ and R₃, in ortho position one with respect to the other, together form an optionally unsaturated chain made by 3 or 4 groups selected among CR'''R^{IV}, CO, S, O and NR^{V}, wherein R"' is hydrogen atom or a C₁-C₄ alkyl group, R^{IV} is a hydrogen atom, a C₁-C₄ alkyl group or an amino group and R^{V} is a hydrogen atom or a C₁-C₄ alkyl group; or R"' together with a vicinal R"' or R^{V} constitutes a single bond or R^{V} together with a vicinal R"' or R^{V} constitutes a single bond;
Y is S,O, N(H)CO, CO(H)N or N(H);
X is N(H), O, S, SO, SO₂, CO or a single bond;
the asterisk marks an asymmetric carbon atom;
and pharmaceutically acceptable salts thereof.

2. A compound according to claim 1 wherein R' is a hydrogen atom, R and R" are OH groups.

3. A process for the preparation of a compound according to claim 1 comprising the reduction of a compound of formula 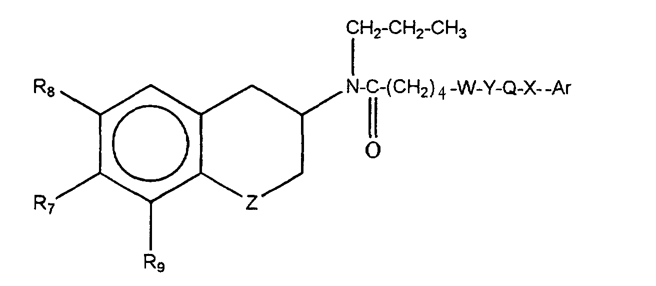 wherein Z, Y, X, and Ar have the meanings already reported in claim 1;
R₇, R₈ and R₉ are hydrogen atoms or OA groups wherein A is a hydrogen atom or a protective group selected among methyl, benzyl, benzoyl and 4-methoxybenzoyl, provided that at least one among R₇, R₈ and R₉ is a hydrogen atom but R₇, R₈ and R₉ are not all contemporaneously hydrogen atoms and R₈ and R₉ are not both contemporaneously OA groups;
W is a CH₂ or CO group;
Q is a group of formula
CO-(CH₂)ₙ₋₁-CHR₁
wherein R₁ has the meanings already reported in claim 1, when n is 1, 2, 3 or 4; or Q is a CHR₁ or CO group, wherein R₁ has the meanings already reported in claim 1, when n is 0.

4. A pharmaceutical composition containing a therapeutically effective amount of a compound according to claim 1 in admixture with a suitable carrier.

5. A pharmaceutical composition according to claim 4 for the treatment of cardiovascular diseases.

## Patentansprüche

1. Eine Verbindung der Formel 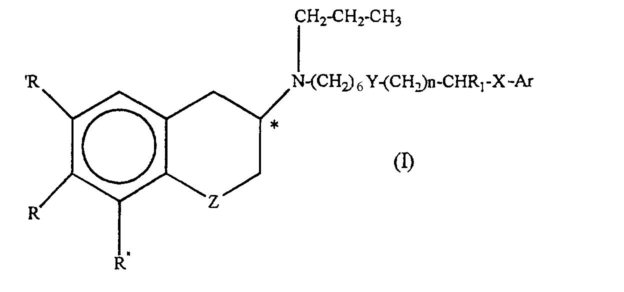 , wobei
Z O, S, SO oder SO₂ ist;
R, R' und R'' Wasserstoffatome oder OH-Gruppen sind, vorrausgesetzt, daß wenigstens ein Rest von R, R' und R'' ein Wasserstoffatom ist aber R, R' und R'' nicht alle gleichzeitig Wasserstoffatome und R' und R" nicht beide gleichzeitig OH-Gruppen sind;
n eine natürliche Zahl ausgewählt aus 0, 1, 2, 3 und 4 ist;
R₁ ein Wasserstoffatom oder eine C₁-C₄-Alkylgruppe ist;
Ar ein Phenyl mit der Formel 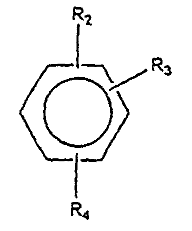 ist, wobei
R₂, R₃ und R₄ gleichsam oder verschieden voneinander Wasserstoff, OH, Halogen, C₁-C₄-Alkyl, C₁-C₄-Alkoxy, Nitro, C₁-C₄-Alkylthio, NH₂, mono- oder di-C₁-C₄-Alkylamino, SH, C₁-C₄-Alkylsulphonyl, C₁-C₄-Alkoxycarbonyl, NHCHO, C₁-C₄-Alkylcarbonylamino, NHCONH₂, C₁-C₄-Alkylsulphonylamino, C₁-C₄-Alkylaminosulphonyl, SO₂NH₂, NHSO₂NH₂, COOH, SO₃H, CONH₂, CH₂OH oder Phenyl sind; oder
R₂ und R₃, in ortho-Position zueinander gemeinsam eine wahlweise ungesättigte Kette bilden, die aus 3 oder 4 Gruppen ausgewählt aus CR'"R^{IV}, CO, S, O und NR^{V} besteht, wobei
R''' ein Wasserstoffatom oder eine C₁-C₄-Alkylgruppe ist,
R^{IV} ein Wasserstoffatom, eine C₁-C₄-Alkylgruppe oder eine Aminogruppe ist und
R^{V} ein Wasserstoffatom oder eine C₁-C₄-Alkylgruppe ist;
oder
R''' zusammen mit einem vincinalen R''' oder R^{V} eine Einfachbindung bildet oder R^{V} zusammen mit einem vincinalen R''' oder R^{V} eine Einfachbindung bildet;
Y S, O, NHCO, COHN oder NH ist;
X NH, O, S, SO, SO₂, CO oder eine Einfachbindung ist;
wobei der Stern ein asymmetrisches Kohlenstoffatom markiert;
und pharmazeutisch akzeptable Salze davon.

2. Eine Verbindung nach Anspruch 1, wobei R' ein Wasserstoffatom ist und R und R" OH-Gruppen sind.

3. Ein Verfahren für die Herstellung einer Verbindung nach Anspruch 1, das die Reduktion einer Verbindung mit der Formel 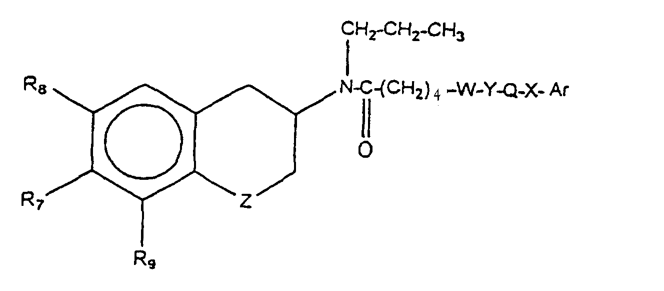 umfaßt, wobei
Z, Y, X und Ar die Bedeutungen haben, wie sie bereits in Anspruch 1 aufgeführt sind;
R₇, R₈ und R₉ Wasserstoffatome oder OA-Gruppen sind, wobei A ein Wasserstoffatom oder eine Schutzgruppe, ausgewählt aus Methyl, Benzyl, Benzoyl und 4-Methoxybenzoyl ist, vorrausgesetzt daß wenigstens ein Rest von R₇, R₈ und R₉ ein Wasserstoffatom ist aber R₇, R₈ und R₉ nicht alle gleichzeitig Wasserstoffatome und R₈ und R₉ nicht beide gleichzeitig OA-Gruppen sind;
W eine CH₂- oder OH-Gruppe ist;
Q eine Gruppe mit der Formel
CO-(CH₂)ₙ₋₁-CHR₁
ist, wobei
R₁ die Bedeutung hat, wie bereits in Anspruch 1 dargestellt, wenn n gleich 1, 2, 3 oder 4 ist;
oder Q eine CHR₁-oder CO-Gruppe ist, wobei R₁ die Bedeutung hat, wie bereits in Anspruch 1 dargestellt, wenn n gleich 0 ist.

4. Eine pharmazeutische Zusammensetzung, die eine therapeutisch effektive Menge einer Verbindung nach Anspruch 1 als Zusatz zu einer geeigneten Trägersubstanz enthält.

5. Eine pharmazeutische Zusammensetzung nach Anspruch 4 für die Behandlung von kardiovaskulären Krankheiten.

## Revendications

1. Composé de formule : 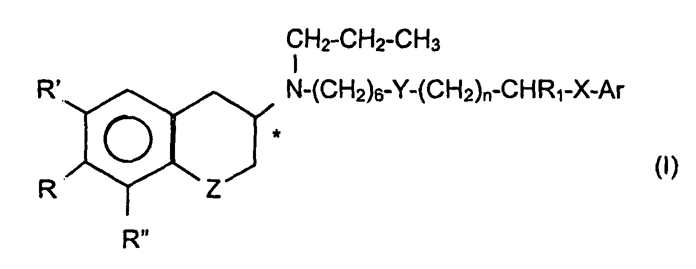 dans laquelle :
Z est O, S, SO ou SO₂;
R, R' et R" sont des atomes d'hydrogène ou des groupes OH, pour autant qu'au moins un parmi R, R' et R" soit un atome d'hydrogène mais que R, R' et R" ne soient pas tous simultanément des atomes d'hydrogène et R' et R" ne soient pas tous deux simultanément des groupes OH;
n est un nombre entier choisi parmi 0, 1, 2, 3 et 4;
R¹ est un atome d'hydrogène ou un groupe alkyle en C₁-C₄;
Ar est un groupe phényle de formule : 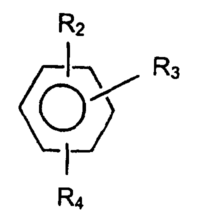 dans laquelle :
R₂, R₃ et R₄, identiques ou différents, sont de l'hydrogène, OH, un halogène, un groupe alkyle en C₁-C₄, alcoxy en C₁-C₄, nitro, alkylthio en C₁-C₄, NH₂, mono- ou dialkylamino en C₁-C₄, SH, alkylsulfonyle en C₁-C₄, alcoxycarbonyle en C₁-C₄, NHCHO, alkylcarbonylamino en C₁-C₄, NHCONH₂, alkylsulfonylamino en C₁-C₄, alkylaminosulfonyle en C₁-C₄, SO₂NH₂, NHSO₂NH₂, COOH₂, SO₃H, CONH₂, CH₂OH ou phényle; ou bien
R₂ et R₃, en position ortho l'un par rapport à l'autre, forment ensemble une chaîne éventuellement insaturée constituée de 3 ou 4 groupes choisis parmi CR"'R^{IV}, CO, S, O et NR^{V}, dans lesquels R"' est un atome d'hydrogène ou un groupe alkyle en C₁-C₄, R^{IV} est un atome d'hydrogène, un groupe alkyle en C₁-C₄ ou un groupe amino et R^{V} est un atome d'hydrogène ou un groupe alkyle en C₁-C₄; ou bien R"' en même temps qu'un R"' ou R^{V} voisin constitue une simple liaison ou R^{V} en même temps qu'un R"' ou R^{V} voisin constitue une simple liaison;
Y est S, O, N(H)CO, CO(H)N ou N(H);
X est N(H), O, S, SO, SO₂, CO ou une simple liaison;
l'astérisque indique un atome de carbone asymétrique;
et les sels pharmaceutiquement acceptables de celui-ci.

2. Composé suivant la revendication 1, dans lequel R' est un atome d'hydrogène, R et R" sont des groupes OH.

3. Procédé de préparation d'un composé suivant la revendication 1, comprenant la réduction d'un composé de formule: 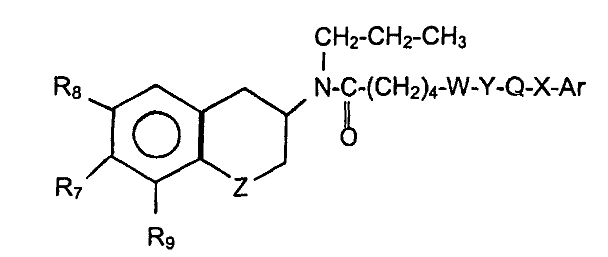 dans laquelle Z, Y, X et Ar ont les significations déjà rapportées dans la revendication 1;
R₇, R₈ et R₉ sont des atomes d'hydrogène ou des groupes OA dans lesquels A est un atome d'hydrogène ou un groupe protecteur choisi parmi les groupes méthyle, benzyle, benzoyle et 4-méthoxybenzoyle, pour autant qu'au moins un parmi R₇, R₈ et R₉ soit un atome d'hydrogène mais que R₇, R₈ et R₉ ne soient pas tous simultanément des atomes d'hydrogène et R₈ et R₉ ne soient pas tous deux simultanément des groupes OA;
W est un groupe CH₂ ou CO;
Q est un groupe de formule CO-(CH₂)ₙ₋₁-CHR₁,
dans laquelle R₁ a les significations déjà rapportées dans la revendication 1, lorsque n est égal à 1, 2, 3 ou 4; ou bien Q est un groupe CHR₁ ou CO, dans lequel R₁ a les significations déjà rapportées dans la revendication 1, lorsque n est égal à 0.

4. Composition pharmaceutique contenant une quantité thérapeutiquement efficace d'un composé suivant la revendication 1, en mélange avec un support approprié.

5. Composition pharmaceutique suivant la revendication 4, pour le traitement de maladies cardio-vasculaires.
